# FASCICULE DE BREVET EUROPEEN

(11) **EP 1 639 139 B1**
(45) Date de publication et mention de la délivrance du brevet: **27.07.2011**
(21) Numéro de dépôt: 04767370.2
(22) Date de dépôt: 17.06.2004
(51) Int. Cl.: C12Q 1/68

(54) **METHODES DE DETECTION DE LA MALADIE D'ALZHEIMER**
VERFAHREN ZUR DIAGNOSE UND PROGNOSE VON MORBUS ALZHEIMER
METHODS FOR THE DIAGNOSIS AND PROGNOSIS OF ALZHEIMER'S DISEASE

(30) Priorité: 20.06.2003 FR 0307501
(43) Date de publication de la demande: 29.03.2006
(73) Titulaire: Aventis Pharma S.A., 92160 Antony (FR)
(72) Inventeur: MACE, Sandrine, F-78350 JOUY-EN-JOSAS (FR); RICARD, Sylvain, F-75013 PARIS (FR); COUSIN, Emmanuelle, F-75011 PARIS (FR); PRADIER, Laurent, F-91370 VERRIERES (FR); BENAVIDES, Jesus, F-92290 CHATENAY-MALABRY (FR); DELEUZE, Jean-François, F-77380 COMBS-LA-VILLE (FR)
(74) Mandataire: Bouvet, Philippe
(86) Numéro de dépôt international: PCT/FR2004/001509
(87) Numéro de publication internationale: WO 2004/113568

(56) Documents cités:
- WO-A-01/14414
- WO-A-02/064781
- WO-A-02/101392
- WO-A2-02/08407
- PALEACU D ET AL: "DONEPEZIL FOR THE TREATMENT OF BEHAVIORAL SYMPTOMS IN PATIENTS WITH ALZHEIMER'S DISEASE" CLINICAL NEUROPHARMACOLOGY, RAVEN PRESS, NEW YORK, NY, US, vol. 25, no. 6, novembre 2002 (2002-11), pages 313-317, XP009027622 ISSN: 0362-5664
- DATABASE EBI [Online] Homo sapiens ABC transporter ABCA2 gene, exon 14 1 mars 2001 (2001-03-01), XP002273183 Database accession no. AF327671 -& KAMINSKI W.E. ET AL.: "COMPLETE CODING SEQUENCE, PROMOTER REGION, AND GENOMIC STRUCTURE OFTHE HUMAN ABCA2 GENE AND EVIDENCE FOR STEROL-DEPENDENT REGULATION IN MACROPHAGES" BIOCHEM. BIOPHYS. RES. COMM., vol. 281, no. 1, 16 février 2001 (2001-02-16), pages 249-258, XP000990418
- DATABASE SNP [Online] NCBI, US; 6 septembre 2000 (2000-09-06), XP002273184 Database accession no. RS908832
- VULEVIC BOJANA ET AL: "Cloning and characterization of human adenosine 5'-triphosphate-binding cassette, sub-family A, transporter 2 (ABCA2)" CANCER RESEARCH, AMERICAN ASSOCIATION FOR CANCER RESEARCH, BALTIMORE, MD, US, vol. 61, no. 8, 15 avril 2001 (2001-04-15), pages 3339-3347, XP002247551 ISSN: 0008-5472
- DATABASE WPI Section Ch, Week 200223 Derwent Publications Ltd., London, GB; Class B04, AN 2002-179907 XP002273118 -& WO 02/08424 A (BANYU PHARM CO LTD) 31 janvier 2002 (2002-01-31)
- WOLLMER M A ET AL: "ABCA1 modulates CSF cholesterol levels and influences the age at onset of Alzheimer's disease" NEUROBIOLOGY OF AGING, TARRYTOWN, NY, US, vol. 24, no. 3, mai 2003 (2003-05), pages 421-426, XP002237201 ISSN: 0197-4580
- DATABASE GENBANK [Online] NCBI; Homo sapiens ATP-binding cassette, sub-family A 6 septembre 2001 (2001-09-06), XP002273185 Database accession no. NM_001606
- CHEN ZHIJIAN J ET AL: "Association of ABCA2 expression with determinants of Alzheimer's disease" FASEB JOURNAL, vol. 18, no. 7, mai 2004 (2004-05), XP002315438 ISSN: 0892-6638

## Description

La présente invention se rapporte à des méthodes de diagnostic ou de pronostic de la maladie d'Alzheimer. Elle concerne également les kits de diagnostic ou de pronostic de la maladie d'Alzheimer.

La maladie d'Alzheimer est une maladie neurodégénérative qui affecte une large proportion de la population âgée. Cette maladie est caractérisée sur le plan clinique par la perte des fonctions cognitives, et sur le plan neuropathologique par la présence dans le cerveau de dépôts neurofibrillaires intracellulaires et de dépôts extracellulaires du peptide β-amyloïde (Aβ) formant les plaques amyloïdes. Les plaques amyloïdes sont majoritairement composées des peptides Aβ à 40 ou 42 acides aminés qui sont générés par un processus protéolytique de la protéine précurseur du peptide β-amyloïde (APP). Les dépôts extracellulaires d'Aβ sont spécifiques de la maladie d'Alzheimer. Ils représentent la caractéristique précoce et invariable de toutes les formes de la maladie d'Alzheimer, incluant les formes familiales.

Les formes familiales de la maladie apparaissent de manière relativement précoce (entre 40 et 60 ans). Elles semblent dues, au moins en partie, à des mutations dans le gène de l'APP et dans les gènes de la préséniline-1 (PS1) et de la préséniline-2 (PS2). Les mutations dans ces trois gènes induisent des changements dans la protéolyse de l'APP, conduisant à une surproduction d'Aβ et à l'apparition précoce de la pathologie et des symptômes qui sont similaires à ceux des formes sporadiques de la maladie d'Alzheimer.

Un lien entre le cholestérol et la maladie d'Alzheimer a également été établi à partir d'études épidémiologiques et de résultats d'études biochimiques et de biologie cellulaire récentes (voir revue Hartmann, T. (2001) TINS 24 : S45-48). Un taux de cholestérol élevé à l'âge adulte ainsi qu'une tension artérielle élevée accroissent significativement le risque de maladie d'Alzheimer (Kivipelto et al., 2001 Br Med J. 322 : 1447).

A l'opposé, un risque très diminué est enregistré dans les populations sous traitement avec les agents hypocholestérolémiants de type statines (Wolozin et al. (2000) Arch Neurol. 57 : 1439 ; Jick et al. (2000) Lancet 356 : 1627).

Le lien moléculaire semble avoir été récemment établi. *In vitro* et *in vivo*, un taux de cholestérol élevé augmente la production du peptide A-β et accélère l'apparition d e p laques amyloides (Sparks et al. (1994) Exp. Neurol. 126 : 88-94 ; Refolo et al. (2000) Neurobiol. Dis. 7 : 321-331 ; Puglielli et al.(2001) Nat. Cell Biol. 3 : 905 ; Shie et al. (2002). Neuroreport 13 : 455) tandis que les inhibiteurs de la voie de synthèse du cholestérol les diminuent (Simons et al. (1998) PNAS USA 95 : 6460-6464 ; Fassbender et al. (2001) PNAS USA 98 : 5856, Refolo et al., (2001) Neurobiol. Dis. 8 : 890-899).

Malgré des avancées significatives, le corps médical est toujours confronté à un manque de molécules réellement efficaces à l'encontre de la maladie d'Alzheimer. Paleacu et al, Clin. Neuropharm. Vol 25, pp313-317 (2002) décrit notamment l'utilisation d'un certain type de traitement pour les symptômes comportementaux de la maladie d'Alzheimer. Une des raisons de ce manque réside dans la difficulté à trouver des cibles permettant un criblage performant de molécules susceptibles d'exercer une action thérapeutique à l'encontre de cette maladie.

D'autre part la détection précoce de cette maladie apparaît déterminant, afin d'apporter un traitement avant que ne se manifestent les premiers symptômes, hautement invalidants pour les malades.

En outre, du fait des formes variées de cette maladie il apparaît nécessaire de cibler le traitement en fonction d'une part des individus à traiter et d'autre part de la molécule susceptible d'être utilisée pour le traitement. Cette approche pharmacogénomique ou pharmacogénétique apparaît de plus en plus importante.

La protéine ABCA2 est une protéine appartenant à la nombreuse famille des transporteurs à cholestérol de type ABC (ATP binding cassette). Ce transporteur est exprimé spécifiquement dans le cerveau.

Le clonage du gène ABCA2 a été décrit en 2000 par Zhao et al (Biochem. J., 350, 865-872). La séquence a en outre fait l'objet de la demande PCT WO 01/14414, déposée par la société ACTTVEPASS PHARMACEUTICALS. Diverses hypothèses

sont formulées dans cette demande quant au rôle d'ABCA2, mais sans qu'aucun résultat ne vienne les supporter.

La demande de PCT WO 02/064781 évoque quant à elle un lien entre un transporteur ABC et l'expression de la protéine précurseur de l'amyloïde.Divers polymorphismes du gène ABCA2 ont été déjà identifiés. Leurs séquences sont en particulier accessibles dans la base de données dbSNP du NCBI. Le polymorphisme rs908832 est décrit, parmi les différents polymorphismes répertoriés dans cette base.

Ce polymorphisme est caractérisé par un allèle majoritaire dans la population caucasienne qui est une guanine localisée en position 348 sur la séquence SEQ ID N°2 et un allèle minoritaire dans la population caucasienne qui est une adénine localisée en position 348 sur la séquence SEQ ID N°1.

Alternativement, ce polymorphisme mononucléotidique est représenté par les séquences SEQ ID N°3 et SEQ ID N°4 partiellement complémentaires respectivement des SEQ ID N° et SEQ ID N°2. Ce polymorphisme est alors caractérisé par un allèle majoritaire dans la population caucasienne qui est une cytosine localisée en position 201 sur la séquence SEQ ID N°4 et un allèle minoritaire dans la population caucasienne qui est une thymidine localisée en position 201 sur la séquence SEQ ID N°3.

Ce polymorphisme est synonyme, c'est-à-dire qu'il ne modifie pas la séquence de la protéine traduite. Les deux allèles du polymorphisme font partie de codons qui codent pour un acide aspartique. Ce polymorphisme codant est localisé sur l'exon 14 du transcrit présentant la séquence SEQ ID N°5 en position 2185.

De manière surprenante, la demanderesse a montré que les sujets présentant l'allèle minoritaire du polymorphisme rs908832 du gène ABCA2 ont un risque accru de développer précocement la maladie d'Alzheimer.

Cette découverte est particulièrement importante car elle apporte pour la première fois, à la connaissance de la demanderesse, la preuve d'un lien entre la protéine ABCA2, et en particulier un polymorphisme du gène codant pour cette protéine, et un état pathologique, tel que la maladie d'Alzheimer.

Elle permet de confirmer et/ou de pronostiquer la sévérité de l'atteinte chez les patients pour lesquels la maladie d'Alzheimer a déjà été diagnostiquée ainsi que l'efficacité probable des traitements envisagés, ou encore de pronostiquer les risques d'apparition de la maladie chez des sujets ne présentant pas de symptômes de la maladie d'Alzheimer incluant les individus apparentés à des patients dont le diagnostic a été confirmé.

Elle permet en outre de confirmer un rôle fonctionnel majeur de ABCA2 dans la physiopathologie de la maladie d'Alzheimer , justifiant de mettre en oeuvre des tests de criblage de molécules susceptibles d'exercer une action stimulatrice ou inhibitrice sur l'activité de ABCA2 en vue d'application thérapeutique à l'encontre de la maladie d'Alzheimer.

La présente invention a ainsi pour premier objet une méthode de diagnostic ou de pronostic la maladie d'Alzheimer chez un sujet. Ladite méthode comprend au moins une étape de détection de la présense du polymorphisme rs908832.

Des sujets appropriés peuvent être par exemple:
- les personnes qui ne présentent pas les symptômes de la maladie d'Alzheimer,
- les personnes chez lesquelles le risque de développer la maladie d'Alzheimer a déjà été détecté, mais qui ne présentent pas encore les symptômes de la maladie, et
- les personnes qui ont préalablement été diagnostiqués comme étant atteintes de la maladie d'Alzheimer pour lesquelles une confirmation du diagnostic est souhaitée.

La, ou les étapes de détection, simultanées ou non, de la présence ou de l'absence de l'allèle minoritaire du polymorphisme rs908832 sont effectuées directement ou indirectement par tout moyen approprié à partir d'échantillons biologiques.

La présente invention concerne donc également une méthode de criblage d'échantillons biologiques prélevés sur des sujets, notamment des sujets ne présentant pas de symptômes de la maladie d'Alzheimer, pour détecter les sujets fortement susceptibles de développer la maladie d'Alzheimer. Un tel criblage comprend la recherche, de manière simultanée ou non, dans lesdits échantillons biologiques, de la présence de l'allèle minoritaire du polymorphisme rs908832.

Lesdits échantillons contenant les ADN ou les protéines à identifier peuvent être de diverses origines. Il peut s'agir par exemple d'échantillons de sang, de sperme, de cheveux (avec leurs racines) ou de tout autre échantillon contenant des cellules nucléées. De préférence, les échantillons biologiques analysés sont des échantillons de sang. Dans ce cas, l'ADN à identifier est prélevé dans les leucocytes.

Au sens de la présente invention, on entend par « échantillon biologique » des échantillons directement issus du sujet pour lequel on souhaite un diagnostic ou un pronostic sans autre transformation, ou des échantillons ayant subi une ou plusieurs étapes de préparation de façon à n'en conserver qu'une fraction utile pour les étapes de détection, par exemple un extrait cellulaire brut.

Les techniques permettant de détecter ou d'identifier la présence ou l'absence des ADN portant l'allèle minoritaire d u polymorphisme r s908832 peuvent être d es combinaisons des techniques de Réaction de Polymérisation en Chaîne (« PCR »),), d'hybridation, de transfert de Southem, de digestion par des nucléases, de Polymorphisme de Longueur des Fragments de Restriction (« RFLP »), et/ou de séquençage direct des produits de la PCR. Toutes ces techniques sont connues de l'homme du métier.

D'une manière générale, toutes les techniques d'identification des ADN portant l'allèle minoritaire du polymorphisme rs908832 qui peuvent être employées dans le cadre de la présente invention comprennent une étape préalable de collecte du ou des échantillons biologiques contenant les ADN à identifier et une étape d'extraction de l'ADN génomique selon les techniques standard bien connues de l'homme du métier, par exemple selon la méthode de Smith et al. (The Lancet, 1992, 339, pp. 1375-7).

Une telle méthode d'identification peut comprendre :
a) l'extraction de l'ADN dudit sujet,
b) l'amplification dudit ADN isolé à l'aide d'amorces capables d'amplifier les séquences correspondant a chacun des allèles du polymorphisme rs908832 du gène ABCA2, et
c) la détermination d'au moins l'un des allèles du polymorphisme rs908832 du gène ABCA2 dans l'ADN amplifié.

Selon un mode de mise en oeuvre avantageux, on utilise la technique de PCR. Ainsi l'étape b) comprend avantageusement une étape de réaction de polymérisation en chaîne. Cette technique consiste tout d'abord à synthétiser des oligonucléotides complémentaires de la séquence des régions qui délimitent le segment d'ADN à amplifier (amorces). Ces oligonucléotides servent d'amorces à la DNA polymérase. Puis, on entreprend les étapes de dénaturation par la chaleur (92-95°C) pour séparer les deux brins d'ADN, d'hybridation avec les deux amorces spécifiques grâce à un abaissement de la température (50-55°C) et d'extension des amorces avec une DNA polymérase à 70-72°C.

De telles amorces permettant l'amplification de chacun des allèles du polymorphisme rs908832 du gène ABCA2, et en particulier de l'allèle minoritaire, ainsi que leurs séquences complémentaires, constituent d'autres objets de la présente demande. Avantageusement elles présentent des séquences respectives telles que le nombre de bases entre leurs points d'hybridation respectifs sur la molécule d'ADN, est compris entre 25 et 2500 paires de bases, et préférentiellement entre 100 et 500 paires de bases.

Avantageusement de telles amorces présentent entre environ 15 et 30 nucléotides contigus de la séquence SEQ ID N°3 ou de la séquence SEQ ID N°4. De manière préférentielle il s'agit d'un couple d'amorces présentant les séquences SEQ ID N°6 et SEQ ID N° 7.

Selon un mode de mise en oeuvre avantageux, la présente demande a pour objet une méthode caractérisée en ce que l'ADN amplifié portant l'allèle minoritaire du polymorphisme rs908832 est distingué de l'ADN amplifié ne portant pas cet allèle par hybridation spécifique de deux sondes, chacune d'elle étant spécifique d'une des deux formes du polymorphisme. Ces sondes, ainsi que leurs séquences complémentaires, constituent d'autres objets de la présente demande.

Avantageusement ces sondes sont constitués respectivement d'environ 12 à 17 nucléotides contigus de la séquence SEQ ID N°3 ou SEQ ID N°4. De manière préférentielle elles présentent respectivement les séquences SEQ ID N°8 et SEQ ID N°9.

L'ADN amplifié portant l'allèle minoritaire du polymorphisme rs908832 peut aussi être distingué de l'ADN amplifié ne portant pas cet allèle par la technique utilisant l'activité 5' nucléase de l'ADN polymérase I (TaqMan).

Selon un mode de mise en oeuvre avantageux, la présente demande a pour objet une méthode caractérisée en ce que l'ADN amplifié portant le polymorphisme rs908832 est distingué de l'ADN amplifié ne portant pas ce polymorphisme par analyse du polymorphisme des fragments de restriction (RFLP). Avantageusement les fragment de restriction on été obtenus par digestion de l'ADN amplifié à l'aide d'une enzyme de restriction avant migration en gel d'agarose, transfert sur membrane par la technique de Southern et hybridation.

Pour obtenir à la fois une plus grande sensibilité et une meilleure spécificité, il est également possible d'effectuer deux PCR successives en utilisant deux couples d'amorces différents (« nested PCR ») : un premier couple d'amorces externes qui permet d'obtenir un fragment d'ADN amplifié comme en PCR classique et un second couple d'amorces internes pour amplifier le fragment d'ADN obtenu de la première PCR.

Afin de déterminer l'empreinte génétique de la région souhaitée, les fragments d'ADN obtenus par PCR peuvent également être séparés par électrophorèse selon leur taille et visualisés grâce au BET (bromure d'éthidium) et aux rayons ultraviolets.

Une possibilité particulièrement intéressante dans le cas présent consiste à effectuer une PCR avec un première amorce ayant à son extrémité 3' la séquence de nucléotides mutée, et une seconde amorce ayant à son extrémité 3' la séquence de nucléotides sauvage. La différence de température de dénaturation dans ces deux cas, et en conséquence d'efficacité d'amplification, permet de distinguer l'ADN mutant de l'ADN sauvage.

Selon une autre alternative, les fragments d'ADN amplifiés sont directement identifiés par transfert en point (technique de Dot Blot) qui consiste à déposer un échantillon des fragments d'ADN produits de la PCR sur un filtre de nylon, à effectuer la dénaturation des fragments d'ADN, leur hybridation avec une sonde spécifique radioactive et un lavage pour éliminer l'excès de produit radioactif non-fixé suivi d'un autoradiogramme. La révélation peut également être faite par d'autres moyens grâce à l'emploi de sondes spécifiques comportant un marqueur autre que radioactif, par exemple un colorant ou encore un marqueur fluorescent.

Selon une autre alternative, il est également possible de détecter la présence ou l'absence de mutations des ADN comprenant le polymorphisme rs908832 par séquençage direct de tout ou partie des fragments d'ADN amplifiés. Un tel procédé consiste à déterminer la séquence nucléotidique au niveau du polymorphisme rs908832. Le séquençage peut être effectué par toute méthode connue de l'homme de l'art, par exemple par la méthode d e S anger ou bien par la méthode de Maxam et Gilbert.

Selon une autre alternative, la détection de la présence ou l'absence des ADN portant le polymorphisme rs908832 peut être effectuée en utilisant la technique de transfert de Southem (Southem Blotting) qui consiste à effectuer une électrophorèse des fragments d'ADN obtenus après une attaque par une ou plusieurs enzymes de restriction. Le gel est ensuite dénaturé et un transfert est opéré sur une membrane de nylon. Cette membrane est destinée à être hybridée avec une sonde spécifique. Après lavage pour éliminer l'excès de produit radioactif non fixé, le film est appliqué sur la membrane. Il peut ainsi être décelé une ou plusieurs bandes correspondant aux fragments d'ADN reconnus par la sonde.

Il est également possible d'utiliser la technique de la RFLP associée à la technique de Southem Blotting et/ou de PCR pour détecter la présence ou l'absence du polymorphisme rs908832. La RFLP permet de comparer les ADN de différents individus et de rechercher si des mutations ponctuelles faisant apparaître ou disparaître des sites de restriction se sont produites. Deux ADN de séquence identique traitées par des enzymes de restriction donneront des fragments identiques, et les Southern Blot obtenus avec ces fragments seront donc identiques. Au contraire, si un site de restriction a disparu ou bien est apparu à la suite d'une mutation, les fragments n'auront plus des tailles identiques ce qui sera visible sur les autoradiogrammes. Il en est de même si un nouveau site de restriction est apparu à la suite d'une mutation.

Il est encore possible de détecter le polymorphisme rs908832 du gène ABCA2 en déterminant le nucléotide en position 2185 du transcrit codant pour la protéine ABCA2.

Toutes les techniques décrites ci-avant sont bien connues de l'homme de l'art. Toute autre technique connue et également appropriée pour la détection de la présence ou de l'absence du polymorphisme rs908832 peut être employée. On peut à ce titre par exemple citer les techniques de « Ligase Chain Reaction », « Strand Displacement Amplification », « Transcription-based Amplification » etc... De façon avantageuse, lesdites étapes de détection sont effectuée par PCR, RFLP, Southem Blotting et/ou une combinaison de ces techniques, conformément aux méthodes décrites dans la littérature (voir par exemple : Gough et al., Nature, 1990, 347, p.773 ; Kagimoto et al., J. Biol. Chem., 1990, 265, p. 17209 ; Wolf et al., The Lancet, 1990, 336, p. 1452 ; Hayashi et al., Nucleic Acids Res., 1991, 19, p. 4797 ; Daly et al., Pharmacogenetics, 1991, 1, p. 33 ; Spurr et al., Methods Enzymol., 1991, 206, p. 149 ; Armstrong et al., The Lancet, 1992, 339, p. 1017 ; Kurth et al., Am. J. of Med. Genet., 1993, 48, p. 166; McCann et al., J. Neurol. Sci., 1997, 153, p. 50 ; Stroombergen et al., Hum. & Exper. Toxicol., 1999, 18, p. 141).

Toutes ces méthodes de détection sont particulièrement utiles car elles constituent la base permettant de déterminer si un sujet peut être atteint par la maladie d'Alzheimer ou bien présente un risque accru de développer la maladie d'Alzheimer.

Ainsi, un autre objet de la présente invention concerne une méthode d'analyse d'échantillons biologiques prélevés sur un sujet qui consiste à :
a) déterminer le génotype pour le gène ABCA2 dudit sujet, et
b) convertir les données obtenues en a) afin de pronostiquer le risque dudit sujet de développer la maladie d'Alzheimer et l'efficacité de traitements thérapeutiques envisageables pour cette maladie.

Des trousses, ou kits, de diagnostic ou de pronostic de la maladie d'Alzheimer chez un sujet sont aussi décrits.

De tels kits peuvent se présenter sous forme d'un emballage compartimenté de façon à recevoir différents récipients tels que par exemple des ampoules ou des tubes. Chacun de ces récipients comprend les différents éléments nécessaires pour effectuer la détection de la présence ou de l'absence de l'ADN portant le polymorphisme rs908832.

Lesdits éléments permettant d'effectuer la ou les réactions de détection sont choisis parmi ceux décrits ci-avant. Il peut s'agir par exemple :
- d'un couple d'amorces hybridant une région définie du gène ABCA2 et éventuellement les moyens nécessaires à la réalisation d'une réaction d'amplification, ou
- de sondes oligonucléotidiques, éventuellement immobilisées sur un support et comprenant un marqueur détectable, et éventuellement les réactifs nécessaires à la réalisation d'une réaction d'hybridation.

La présente invention concerne en outre un animal transgénique non humain dans le génome duquel est inséré au moins une séquence d'ADN exogène portant l'allèle minoritaire du polymorphisme rs908832 de telle sorte que la fonction du gène ABCA2 est modifiée.

On entend par animal transgénique tout animal non-humain présentant une modification artificielle de son génome. La modification du génome peut résulter d'une altération ou d'une modification de un ou plusieurs gènes par « knock-in » ou par « knock-out » (inactivation/modification de gènes par recombinaison homologue) ou la surexpression du gène humain sous le contrôle de promoteurs spécifiques des types cellulaires neuronaux (tel que ThyI, PDGF ou prion) ou glial (tel que GFAP) dans la souris. Cette modification peut être due à l'action d'agents altérants ou mutagènes classiques ou bien effectuée par insertion stable d'une cassette d'expression permettant l'expression d'un gène hybride. En particulier, on peut par exemple opérer selon des méthodes identiques ou analogues à celles décrites dans les demandes WO 01/02552 ou encore WO 01/13176.

La modification du génome peut également résulter d'une insertion de gène(s) ou d'un remplacement de gène(s) dans sa (leur) forme sauvage ou mutée.

Les modifications sont avantageusement effectuées sur des cellules souches reproductrices.

A l'heure actuelle, la modification du génome en utilisant la technologie « knock-in » et « knock-out » est limitée à la souris comme modèle du fait que seules les cellules souches (dites cellules « ES ») ayant la capacité de coloniser la lignée germinale issue de l'embryon de souris sont disponibles. Lorsque les lignées cellulaires ES seront établies pour d'autres espèces, ces technologies pourront facilement être appliquées par l'homme de l'art à ces autres espèces pour générer des modèles KO et/ou KI. De plus, les approches basées sur l'utilisation d'oligonucléotides (ADN, ARN ou hybrides) seuls ou liés à des enzymes modifiant l'ADN/ARN, peuvent être utilisées pour introduire une modification/mutation définie à un locus prédéterminé dans le génome. Même des irradiations ou des mutagènes chimiques qui induisent des modifications aléatoires dans le génome peuvent être employées s'ils sont combinés avec un jeu efficace de marqueurs biologiques (phénotype) et avec une procédure de clonage positionnel à haut débit.

L'approche la plus directe pour la modification du génome d'animaux de laboratoire (souris, rats, vaches, cochons, moutons...) est cependant l'intégration aléatoire de transgènes par microinjection d'ADN linéarisé dans un ou deux pronucléi d'ovocytes fertilisés à l'étape de cellule unique (de préférence pour éviter la génération d'animaux chimériques bien que l'injection au stade deux cellules ou plus puisse également être mise en oeuvre). En règle générale, un transgène est composé de deux parties : les éléments de régulation qui imposent le contrôle spatio-temporel de l'expression de l'ARN codé par l'ADN, et ledit ADN juxtaposé (ADNc ou fragment génomique). Ces deux éléments (l'élément de régulation et l'ADN codant pour la protéine désirée) peuvent être homologues ou bien hétérologues du génome cible. Les animaux transgéniques concernés sont d'une manière générale choisis parmi les mammifères non-humains. Il peut s'agir par exemple de murins, à savoir les souris, les rats et les cobayes, de lapins, de chats, de chiens, d'ovins, ou encore de bovins. De préférence, il s'agit de murins, de rats ou de lapins obtenus selon les techniques classiques de transgenèse. D'autres objets de la présente invention sont des lignées de cellules souches et lignées de cellules différenciées à partir de ces lignées de cellules souches, dans le génome desquels est insérée au moins une séquence d'ADN exogène génomique portant l'allèle minoritaire du polymorphisme rs908832.

Succinctement, l'obtention des animaux transgéniques, des lignées de cellules souches et lignées de cellules différenciées selon l'invention consiste en un procédé mettant en oeuvre la génération d'animaux transgéniques par insertion par recombinaison homologue d'un ADN exogène génomique codant pour la protéine ABCA2 dans le gène correspondant de l'animal (l'insertion du transgène est ciblée immédiatement après le promoteur du gène de l'animal de façon à imposer le profil d'expression correct au t ransgène et à empêcher l'expression du gène endogène de l'animal : knocked out) ou bien par insertion de la mutation spécifique décrite dans la présente invention correspondant à une isoforme du gène ABCA2 humain dans le gène endogène de l'animal (la modification est faite par recombinaison homologue dans les cellules souches : knocked in)., ou bien par surexpression du gène ABCA2 portant l'allèle minoritaire du polymorphisme rs908832.

Dans le cas des souris, ces animaux pourront être avantageusement croisées avec des souris transgéniques portant le gène APP humain arborant des mutations de type Alzheimer et qui développent des plaques amyloides. Les animaux doubles transgéniques ainsi obtenus reproduisent le génotype observé chez les patients atteints par la maladie d'Alzheimer ou à risque. Le contrôle du génotype du polymorphisme rs908832 de l'animal nouveau-né ainsi obtenu peut être effectué en utilisant les techniques déjà décrites ci-avant, notamment en utilisant une réaction d'amplification (PCR) et/ou de Southern.

De tels animaux transgéniques sont particulièrement intéressants car ils fournissent un modèle avantageux pour la compréhension de la maladie d'Alzheimer qui reproduit très fidèlement les caractéristiques de la maladie d'Alzheimer. En comparaison des modèles connus, ce modèle permet notamment la mise en évidence de composés particulièrement adaptés au traitement de la maladie d'Alzheimer, notamment telle que décrite chez l'homme. Ces composés peuvent être des molécules chimiques, des molécules peptidiques ou protéiques, des anticorps, des molécules chimériques ainsi que des ADNs antisens ou des ribozymes.

Les composés ainsi mis en évidence peuvent être utilisés comme médicament, tels quels ou en association avec un véhicule pharmaceutiquement acceptable afin d'obtenir une composition pharmaceutique. Il peut s'agir e n particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables. Les injections pouvant être réalisées par voie stéréotaxique, topique, orale, parentérale, intranasale, intraveineuse, intramusculaire, sous-cutanée, intraoculaire, transdermique, etc.

La mise en évidence des composés décrits précédemment repose sur la mise en contact, notamment par une administration telle que par exemple une injection, du modèle animal de l'invention avec un composé ou un mélange de composés supposé(s) avoir une action et de mesurer ensuite le ou les effet(s) des composés notamment au niveau cérébral du modèle sur différents changements biochimiques et/ou histologiques.

Outre le fait de pouvoir tester *in vivo* des composés thérapeutiques permettant de prévenir, d'atténuer ou de guérir la maladie d'Alzheimer, ces animaux transgéniques permettent également de disposer d'un modèle animal de la maladie d'Alzheimer utile pour cribler des facteurs environnementaux qui induisent ou accélèrent la pathogenèse, ou encore pour étudier le comportement lors du développement de la maladie et étudier les divers mécanismes biologiques qui sont impliqués, par exemple dans le but de l'étude de nouveaux médicaments ou la détermination des quantités efficaces de médicaments et de la toxicité. Ainsi, un autre objet de la présente invention concerne l'utilisation d'un animal transgénique, de lignées de cellules souches ou de lignées de cellules différenciées tels que définis ci-avant pour tester l'activité de composés ou de méthodes destinées à prévenir et/ou traiter la maladie d'Alzheimer. Un autre objet de l'invention concerne une cellule extraite des animaux transgéniques tels que décrits précédemment ainsi que son utilisation pour la mise en évidence de composés destinés au traitement de la maladie d'Alzheimer.

La mise en évidence de composés décrits précédemment repose sur la mise en contact de cellules extraites du modèle animal de l'invention avec un composé ou un mélange de composés supposé(s) avoir une action et de mesurer ensuite le ou les effets des composés au niveau des cellules entières, dans des homogénats de cellules ou sur une fraction sub-cellulaire, sur différents paramètres tels que la mort cellulaire par exemple.

Outre les dispositions qui précèdent, la présente invention comprend également d'autres caractéristiques et avantages qui ressortiront des exemples et figures qui suivent, et qui doivent être considérés comme illustrant l'invention sans en limiter la portée.

### EXEMPLES

### Exemple 1 : Etude du génotype du polymorphisme rs908832 du gène ABCA2 dans un échantillon de patients

Une collection d'ADNs issus de patients d'origine caucasienne atteints de la maladie d'Alzheimer et d'individus témoins a été utilisée, après leur consentement éclairé.

La fréquence des polymorphismes a été déterminée par génotypage d'un échantillon de 47 individus d'un panel commercialisé par l'institut Coriell (USA).

Dix polymorphismes (dont le polymorphisme r s908832) ont été sélectionnés sur la base de leur localisation dans le gène et de leur fréquence afin d'être génotypés chez les patients atteints de la maladie d'Alzheimer et les témoins.

Les génotypes ont été réalisés par la méthode de 5' nuclease assay (technique de discrimination allélique d'Applied Biosystems, Foster City, USA), puis une analyse statistique de type Chi2 a été réalisée en testant l'association de chacun de ces marqueurs avec la maladie d'Alzheimer (test d'hétérogénéité). Pour l'analyse, des groupes ont été distingués selon de l'âge d'apparition de la maladie ((précoce (avant ou à 65 ans) ou tardive (après 65 ans)) et son origine (sporadique (premier cas connu) ou familiale (d'autres cas déjà connus dans la même famille)).

Les résultas obtenus pour le polymorphisme rs908832 sont les suivants :
- fréquence de l'allèle minoritaire (T) chez les patients (440 génotypes): 7.4
- fréquence de l'allèle minoritaire (T) chez les témoins (519 génotypes): 3.4

Les proportions d'Hardy Weinberg ont été vérifiées chez les patients et les témoins: on n'observe pas d'écart significatif ce qui confirme qu'il n' y a pas d'erreur de génotypage.

Résultats des tests effectués chez les patients présentant une forme précoce de la maladie (avant ou à 65ans):
- 136 cas sporadiques vs 272 témoins:
- test d'hétérogénéité: chi2 (1dd1) = 22.69 p = 2.10⁻⁶
- test allélique (freq(T) vs freq(C)): chi2 (1dd1) = 21.27 p =4.10⁻⁶
- 104 cas familiaux vs 272 témoins:
- test d'hétérogénéité: chi2 (2ddl) = 7.80 p = 0.02
- test allélique (freq(T) vs freq(C)): chi2 (1dd1) =7.27 p = 7.10⁻³

L'odd ratio a été estimé par régression logistique chez les 240 patients présentant la forme précoce (ajustement sur le sexe et le statut APOE-ε4). Le résultat est également significatif : OR = 3.97 IC = [2.23 - 7.09]

Les valeurs obtenues restent significatives (seuil fixé à 0,05) même après correction pour tests multiples par la méthode de Bonferroni.

Ces résultats indiquent que le gène ABCA2 est un gène important dans l'étiologie de la maladie d'Alzheimer.

En terme de risque ce résultat est du même ordre de grandeur que le risque conféré par l'apoliprotéine E4, le facteur de risque le plus fort connu à ce jour pour la maladie d'Alzheimer.

### Exemple 2 : Obtention de souris transgéniques

### 1. Génération d'une souris transgénique exprimant la protéine humaine ABC A2

La mutagenèse de l'ABCA2 humaine est faite via l'utilisation d'un système de mutagenèse *in vitro* comme le Sculptor^{™} (Amersham, France). La région codante de l'ABCA2, est sous-clonée dans un vecteur de clonage du type Bluescript (Stratagène) et les mutations sont introduites selon le protocole fourni par le fabricant via l'utilisation d'oligonucléotides contenant la mutation désirée. Les séquences mutées sont à vérifier par analyse de séquence.

### 2. Génération et identification des souris transgéniques ABCA2

Pour la construction du transgène, l'ADN génomique codant pour l'ABCA2 et présentant l'allèle minoritaire du polymorphisme rs908832 humain est sous-clonée dans le polylinker d'un vecteur d'expression transgénique spécifique de certains tissus/types cellulaires comme le THYI (Lüthi et al., J. Neuroscience, 17, pp. 4688-99), , PDGF ou prion pour les types neuronaux, ou GFAP pour les types astrocytaires Un kit de préparation de plasmide (Qiagen) est utilisé pour préparer de l'ADN superenroulé. Pour la microinjection, les séquences du vecteur sont à éliminer par digestion avec une enzyme de restriction définie, laissant intacte la totalité du transgène et le séparant des séquences inutiles du vecteur de clonage. Ensuite, le fragment contenant la cassette d'expression est purifié par électrophorèse sur un gel d'agarose.

Les aliquotes destinés à la microinjection sont dialysés contre un tampon TE (10 mM Tris pH 7,4 ; 0,1 mM EDTA) sur un filtre flottant (Millipore ; type de membrane : VS ; 0,025 µm) puis filtrés (Spin-X ; Costar ; membrane polyacétate ; 0,22 µm). L'ADN est dilué à la concentration finale de 1-2 ng/µl pour la microinjection. Le fragment purifié est injecté dans un des deux pronucléi des ovocytes fertilisés de souris. Les embryons survivants sont immédiatement transplantés dans l'oviducte de mères adoptives (« pseudopregnant »). La présence du transgène chez les nouveau-nés est déterminée soit par PCR soit par une analyse en Southem, en utilisant des sondes/séquences spécifiques. Par l'ensemble de ces analyses, tout réarrangement majeur ou délétions du transgène dans les fondateurs et leur descendance peuvent être exclus.

### 3. Génération d'animaux transgéniques comportant la protéine humaine ABCA2 dans leur génome

On utilise la technologie de recombinaison homologue dans les cellules souches pour introduire le gène ABCA2 présentant l'allèle minoritaire du polymorphisme rs908832 humain à la position prédéfinie désirée dans le gène de la souris. Les amorces et échantillons décrits dans la présente invention peuvent être facilement utilisés pour cribler des librairies génomiques isogéniques de souris (librairies lambda, BAC, YAC ...) afin d'isoler et de cloner le gène correspondant de la souris. Ledit gène de la souris est caractérisé pour son organisation génomique et sa séquence en utilisant les techniques standard connues de l'homme de l'art (cartographie des sites de restriction, séquençage, outils bioanalytiques) afin de définir la place exacte où l'ADN humain doit être inséré pour obtenir le profil d'expression de l'ADN humain désiré tout en interrompant définitivement l'expression du gène murin. Une fois la position exacte identifiée, on construit un vecteur de ciblage standard pour les cellules souches (c'est-à-dire un vecteur ayant des marqueurs pour la sélection des évènements désirés, lesdits marqueurs étant tous bien connus de l'homme de l'art spécialiste du domaine). Rapidement, une cassette de sélection (gène de résistance à un antibiotique) est mise en limite des extrémités 3' et 5' des fragments d'ADN génomique de la souris (2-6 kb) identiques aux extensions 3' et 5' de la séquence du gène murin ABCA2 situé immédiatement après le site d'insertion sélectionné.

Sur la base de la connaissance du gène de la souris, un vecteur de contrôle positif pour le criblage des cellules souches recombinantes est généré (le vecteur reproduit le locus du gène murin une fois l'intégration réussie) a fin d'optimiser et valider la procédure de criblage à haut débit.

Conformément aux procédures standards, l'ADN est purifié pour les essais de ciblage. Le vecteur de ciblage est introduit dans les cellules souches en utilisant des techniques d'électroporation standardisées, après quoi les clones de cellules souches sont soumis à une procédure de criblage séquentiel (antibiotiques) de façon à favoriser les clones de cellules souches portant la recombinaison désirée. En général, le criblage prend environ 2 semaines. Les clones des cellules souches résistants sont criblés par PCR et/ou Southern.

Les clones des cellules souches ayant la recombinaison désirée sans autre modification détectable dans leur génome sont développés de façon à obtenir suffisamment de cellules. Puis, lesdites cellules sont injectées dans des embryons de 3 jours et demi obtenus d'une femelle ayant ovulé naturellement. Les blastocytes survivants (comportant les cellules souches) sont implantés dans une femelle receveuse qui permettra le développement des blastocytes à leur terme et donnera naissance à des souriceaux nouveau-nés composés de cellules provenant des blastocytes hôtes et des clones de cellules souches. Ce type d'animal est appelé « animal chimère ».

Ces chimères (de préférence des mâles puisque la plupart des lignées de cellules souches sont obtenues de souris mâles) sont « mariées » avec des souris de type sauvage de façon à obtenir des animaux hétérozygotes pour la modification. L'élevage d'animaux hétérozygotes entre eux permet de générer des animaux homozygotes.

### 4. Génération d'animaux transgéniques comportant l'allèle minoritaire du polymorphisme rs908832 du gène ABCA2 humain dans leur gène correspondant

De la même façon que décrit précédemment, le gène murin est isolé et caractérisé. Pour ce type de modification, il est essentiel de comparer le gène humain et le gène murin de façon à identifier la position exacte où le point de mutation trouvé chez l'homme doit être introduit dans le gène murin. Une bioanalyse est essentielle à cette étape. A la fin, le vecteur de ciblage est élaboré et assemblé de la même façon que décrit ci-avant. Après recombinaison homologue réussie, rien d'autre que le point de mutation désiré n'a changé dans la séquence codante du gène murin. Certains marqueurs de sélection peuvent rester dans un intron, mais ils n'ont en général aucune influence sur l'expression du gène. Si nécessaire, ils peuvent être éliminés en utilisant une seconde génération de vecteurs de ciblage incluant des éléments de reconnaissance recombinase. Une fois la construction assemblée, les étapes sont identiques à celles de la procédure décrite précédemment.

De façon générale, ces souris transgéniques ABCA2 seront favorablement croisées avec des souris transgéniques exprimant le gène APP portant les mutations des formes familiales de la maladie d'Alzheimer afin de favoriser la déposition de plaques amyloides.

### 5. Neurohistopathologie

### Préparation du tissu cérébral

Les souris doivent être profondément anesthésiées (Pentobarbital: 60 mg/ml/kg i.p., Kétamine: 40 mg/ml/kg i.p.) puis perfusées en transcardiaque avec du sérum physiologique puis du paraformaldéhyde (4% dans du PBS). Les cerveaux sont ensuite prélevés puis postfixés dans la même solution de fixation 24 heures à 4°C. Après la fixation, les cerveaux sont séparés en hémicerveaux droit et gauche puis soumis au protocole classique d'enrobage dans la paraffine.

Les hémicerveaux gauches enrobés dans la paraffine des souris transgéniques et non transgéniques, et également des blocs de tissu de cerveau humain postmortem (cortex frontal) de sujets atteints par la maladie d'Alzheimer et d'un sujet contrôle sont coupés à 6 µm d'épaisseur (coupes sériées), via l'utilisation d'un microtome (LEICA RM 2155, France). Les blocs de tissu correspondants aux hémicerveaux droits des souris transgéniques et non transgéniques sont coupés à une épaisseur de 25 µm.

L'immunoréactivité contre le peptide amyloide Aβ sera détectée comme communément décrit par l'homme de l'art.
## Revendications

1. Méthode de diagnostic ou de pronostic de la maladie d'Alzheimer chez un sujet **caractérisée en ce qu'**elle comprend au moins une étape de détection de la présence du polymorphisme rs908832 du gène ABACA2.

2. Méthode selon la revendication 1, **caractérisée en ce que** lesdites étapes de détection sont effectuées à partir d'échantillons biologiques.

3. Méthode de diagnostic ou de pronostic de la maladie d'Alzheimer chez un sujet selon la revendication 2, **caractérisée en ce que** lesdits échantillons biologiques sont des échantillons contenant des cellules nucléées.

4. Méthode de diagnostic ou de pronostic de la maladie d'Alzheimer chez un sujet selon les revendications 2 ou 3, **caractérisée en ce que** lesdits échantillons biologiques sont des échantillons de sang, de sperme ou de cheveux.

5. Méthode de diagnostic ou de pronostic de la maladie d'Alzheimer chez un sujet selon la revendication 1, **caractérisée en ce que** lesdites étapes de détection de la présence ou de l'absence dés ADN portant le polymorphisme sont effectuées par des techniques de réaction de polymérisation en chaîne (PCR), d'hybridation, de transfert sur membrane par la technique de Southern, de digestion par des nucléases, de polymorphisme de longueur des fragments de restriction (RFLP), ou de séquençage direct, ou leur combinaison.

6. Méthode selon la revendication 5 **caractérisée en ce qu'**elle comprend :
a. l'extraction de l'ADN dudit sujet à partir d'un échantillon biologique,
b. l'amplification dudit ADN isolé à l'aide d'amorces capables d'amplifier la séquence correspondant au polymorphisme rs908832 du gène ABCA2,
c. la détermination de la présence d'au moins l'une des allèles du polymorphisme rs908832 du gène ABCA2 dans l'ADN amplifié.

7. Méthode selon la revendication 6 **caractérisée en ce que** l'étape d'amplification b) comprend une réaction de polymérisation en chaîne (PCR).

8. Méthode selon la revendication 6 **caractérisée en ce que** l'ADN amplifié portant l'allèle minoritaire "T" du polymorphisme rs908832 est distingué de l'ADN amplifié ne portant pas cet allèle par la technique utilisant l'activité 5' nucléase de l'ADN polymérase I (TaqMan).

9. Méthode selon la revendication 6 **caractérisée en ce que** l'ADN amplifié portant l'allèle minoritaire "T" du polymorphisme rs908832 est distingué de l'ADN amplifié ne portant pas ce polymorphisme par analyse du polymorphisme des fragments de restriction (RFLP).

10. Méthode selon la revendication 9 **caractérisée en ce que** les fragments de restriction ont été obtenus par digestion de l'ADN amplifié à l'aide d'une enzyme de restriction avant migration en gel d'agarose, transfert sur membrane par la technique de Southern et hybridation.

11. Méthode selon la revendication 1, **caractérisée en ce que** la détection de la présence du polymorphisme rs908832 du gène ABCA2 comprend la détermination du nucléotide en position 2185 du transcrit codant pour la protéine ABCA2, ledit transcrit présentant la séquence SEQ ID N° 5.

12. Animaux transgéniques non-humains, lignées de cellules souches non-humaines et lignées de cellules différenciées à partir de ces lignées de cellules souches, dans le génome desquels est insérée au moins une séquence d'ADN exogène génomique portant l'allèle minoritaire "T" du polymorphisme rs908832 du gène ABCA2.

13. Animaux transgéniques selon la revendication 12 **caractérisés en ce qu'**ils sont choisis parmi les mammifères non-humains.

14. Procédé d'obtention d'animaux transgéniques, de lignées de cellules souches et de lignées de cellules différenciées à partir de ces lignées de cellules souches, selon l'une des revendications 12 et 13, **caractérisé en ce qu'**il comprend la génération d'animaux transgéniques, à l'exception des procédés consistant ou comprenant le croisement séxué de l'ensemble du génome des animaux, de lignées de cellules souches et de lignées de cellules différenciées à partir de ces lignées de cellules souches, comprenant l'allèle minoritaires du polymorphisme rs908832 du gène ABCA2 humain.

15. Utilisation d'animaux transgéniques, de lignées de cellules souches et de lignées de cellules différenciées à partir de ces lignées de cellules souches, selon l'une des revendications 12 et 13 pour tester l'activité d'agents ou de méthodes destinés à prévenir et/ou traiter la maladie d'Alzheimer.

16. Cellule extraite d'animaux transgéniques tel que décrit dans l'une des revendications 12 et 13.

17. Utilisation d'une cellule selon la revendication 16 pour la mise en évidence de composés destinés au traitement de la maladie d'Alzheimer.

## Claims

1. Method for the diagnosis or for the prognosis of Alzheimer's disease in an individual, **characterized in that** it comprises at least one step of detection of the presence of the rs908832 polymorphism of the ABCA2 gene.

2. Method according to Claim 1, **characterized in that** said detection steps are carried out using biological samples.

3. Method for the diagnosis or for the prognosis of Alzheimer's disease in an individual according to Claim 2, **characterized in that** said biological samples are samples containing nucleated cells.

4. Method for the diagnosis or for the prognosis of Alzheimer's disease in an individual according to Claim 2 or 3, **characterized in that** said biological samples are blood samples, sperm samples or hair samples.

5. Method for the diagnosis or for the prognosis of Alzheimer's disease in an individual according to Claim 1, **characterized in that** said steps of detection of the presence or absence of the DNAs carrying the polymorphism are carried out by techniques of polymerase chain reaction (PCR), of hybridization, of Southern blotting onto membrane, of digestion with nucleases, of restriction fragment length polymorphism (RFLP) or of direct sequencing, or a combination thereof.

6. Method according to Claim 5, **characterized in that** it comprises:
a. extracting the DNA of said individual from a biological sample,
b. amplifying said isolated DNA using primers capable of amplifying the sequence corresponding to the rs908832 polymorphism of the ABCA2 gene,
c. determining the presence of at least one of the alleles of the rs908832 polymorphism of the ABCA2 gene in the amplified DNA.

7. Method according to Claim 6, **characterized in that** the amplification step b) comprises a polymerase chain reaction (PCR) .

8. Method according to Claim 6, **characterized in that** the amplified DNA carrying the minor allele of the rs908832 polymorphism is distinguished from the amplified DNA not carrying this allele by the technique using the 5' nuclease activity of DNA polymerase I (TaqMan).

9. Method according to Claim 6, **characterized in that** the amplified DNA carrying the minor allele of the rs908832 polymorphism is distinguished from the amplified DNA not carrying this polymorphism by restriction fragment polymorphism (RFLP) analysis.

10. Method according to Claim 9, **characterized in that** the restriction fragments have been obtained by digestion of the amplified DNA with a restriction enzyme before migration in agarose gel, Southern blotting onto membrane and hybridization.

11. Method according to Claim 1, **characterized in that** the detection of the presence of the rs908832 polymorphism of the ABCA2 gene comprises the determination of the nucleotide at position 2185 of the transcript encoding the ABCA2 protein, said transcript having the sequence SEQ ID No. 5.

12. Non-human transgenic animals, stem cell lines and lines of cells differentiated from these stem cell lines, into the genome of which is inserted at least one exogenous genomic DNA sequence carrying the minor allele of the rs908832 polymorphism of the ABCA2 gene.

13. Transgenic animals according to Claim 12, **characterized in that** they are chosen from nonhuman mammals.

14. Method for obtaining transgenic animals, stem cell lines and lines of cells differentiated from these stem cell lines, according to either of Claims 12 and 13, **characterized in that** it comprises generating transgenic animals, stem cell lines and lines of cells differentiated from these stem cell lines, comprising the minor allele of the rs908832 polymorphism of the human ABCA2 gene.

15. Use of transgenic animals, of stem cell lines and of lines of cells differentiated from these stem cell lines, according to either of Claims 12 and 13, for testing the activity of agents or of methods intended to prevent and/or treat Alzheimer's disease.

16. Cell extracted from transgenic animals as described in either of Claims 12 and 13.

17. Use of a cell according to Claim 16, for demonstrating compounds intended for the treatment of Alzheimer's disease.

## Patentansprüche

1. Verfahren zur Diagnose oder Prognose von Alzheimer-Krankheit bei einem Individuum, **dadurch gekennzeichnet, dass** es mindestens einen Schritt umfasst, in dem man das Vorliegen des rs908832-Polymorphismus des ABCA2-Gens nachweist.

2. Verfahren nach Anspruch 1, **dadurch gekennzeichnet, dass** die Nachweisschritte an biologischen Proben durchgeführt werden.

3. Verfahren zur Diagnose oder Prognose von Alzheimer-Krankheit bei einem Individuum nach Anspruch 2, **dadurch gekennzeichnet, dass** es sich bei den biologischen Proben um Proben handelt, die Karyozyten enthalten.

4. Verfahren zur Diagnose oder Prognose von Alzheimer-Krankheit bei einem Individuum nach den Ansprüchen 2 oder 3, **dadurch gekennzeichnet, dass** es sich bei den biologischen Proben um Proben von Blut, Sperma oder Haaren handelt.

5. Verfahren zur Diagnose oder Prognose von Alzheimer-Krankheit bei einem Individuum nach Anspruch 1, **dadurch gekennzeichnet, dass** die Schritte zum Nachweis des Vorliegens oder Fehlens von DNA, die den Polymorphismus enthalten, mittels Kettenpolymerisationsreaktions- (PCR-), Hybridisierungstechniken, Membrantransfer durch die Southern-Technik, Nukleasespaltungs-, Restriktionsfragmentlängenpolymorphismus- (RFLP-) oder direkte Sequenzierungstechniken oder deren Kombination durchgeführt werden.

6. Verfahren nach Anspruch 5, **dadurch**-**gekennzeichnet**, dass es Folgendes umfasst:
a. die Extraktion der DNA des Individuums aus einer biologischen Probe,
b. die Amplifikation der isolierten DNA mithilfe von Primern, die die Sequenz amplifizieren können, die dem rs908832-Polymorphismus des ABCA2-Gens entspricht,
c. den Nachweis des Vorliegens von mindestens einem der Allele des rs908832-Polymorphismus des ABCA2-Gens in der amplifizierten DNA.

7. Verfahren nach Anspruch 6, **dadurch gekennzeichnet, dass** der Amplifikationsschritt b) eine Kettenpolymerisationsreaktion (PCR) umfasst.

8. Verfahren nach Anspruch 6, **dadurch gekennzeichnet, dass** die amplifizierte DNA, die das seltenere Allel des rs908832-Polymorphismus enthält, von der amplifizierten DNA, die dieses Allel nicht enthält, durch eine Technik unter Verwendung der 5'-Nuklease-Aktivität der DNA-Polymerase I (TaqMan) unterschieden wird.

9. Verfahren nach Anspruch 6, **dadurch gekennzeichnet, dass** die amplifizierte DNA, die das seltenere Allel des rs908832-Polymorphismus enthält, von der amplifizierten DNA, die diesen Polymorphismus nicht enthält, durch Analyse des Restriktionsfragmentlängenpolymorphismus (RFLP) unterschieden wird.

10. Verfahren nach Anspruch 9, **dadurch gekennzeichnet, dass** die Restriktionsfragmente durch Spaltung der amplifizierten DNA mithilfe eines Restriktionsenzyms vor der Wanderung durch ein Agarosegel, Transfer auf Membran durch die Southern-Technik und Hybridisierung erhalten worden sind.

11. Verfahren nach Anspruch 1, **dadurch gekennzeichnet, dass** der Nachweis des Vorliegens des rs908832-Polymorphismus des ABCA2-Gens die Bestimmung des Nukleotids an Position 2185 des Transkripts, das für das ABCA2-Protein kodiert, umfasst, wobei das Transkript die Sequenz SEQ ID NR: 5 aufweist.

12. Nicht-menschliche transgene Tiere, Stammzelllinien und differenzierte Zelllinien ausgehend von diesen Stammzelllinien, in deren Genom mindestens eine exogene genomische DNA-Sequenz inseriert worden ist, die das seltenere Allel des rs908832-Polymorphismus des ABCA2-Gens enthält.

13. Transgene Tiere nach Anspruch 12, **dadurch gekennzeichnet, dass** sie aus nicht-menschlichen Säugern ausgewählt sind.

14. Verfahren zum Erhalten von transgenen Tieren, Stammzelllinien und differenzierten Zelllinien ausgehend von diesen Stammzelllinien nach einem der Ansprüche 12 und 13, **dadurch gekennzeichnet, dass** man bei dem Verfahren transgene Tiere, Stammzelllinien und differenzierte Zelllinien ausgehend von diesen Stammzelllinien herstellt, die das seltenere Allel des rs908832-Polymorphismus des ABCA2-Gens enthalten.

15. Verwendung von transgenen Tieren, Stammzelllinien und differenzierten Zelllinien ausgehend von diesen Stammzelllinien nach einem der Ansprüche 12 und 13 zum Testen der Aktivität von Mitteln oder Verfahren zur Prävention und/oder Behandlung von Alzheimer-Krankheit.

16. Zelle, die aus transgenen Tieren nach einem der Ansprüche 12 und 13 gewonnen wird.

17. Verwendung einer Zelle nach Anspruch 16 zum Nachweis von Verbindungen für die Behandlung von Alzheimer-Krankheit.


<110> AVENTIS PHARMA S.A.
<120> METHODES DE DETECTION DE LA MALADIE D'ALZHEIMER
<130> PRJ03027
<160> 9
<170> PatentIn version 3.1
<210> 1
   <211> 562
   <212> DNA
   <213> Homo sapiens
<400> 1
   ttcatcacct gcgggtgggc caggggcttg gggcaggccc cggggaggac gccgcccctc 60
   cctgccagcc cgcgcctcca gggagagtcc cggcccgcgc acctccttga gccggtgctc 120
   cttctccgcc acgatgtgct ggatggtcat ggccacggag tagacccagg agatcaccat 180
   gcacagcggc atcatgtgct caatgacaaa caggaagctg cggggaggcc gcgctcaggc 240
   gccactcagc cccagcccca gccccagccc cgggcgccca gcactcactc atcgcgtgtg 300
   tagcaggggt aggggaacat ctgcacgtag ctgcctggct ccaccacatc gtgccccaca 360
   aaagtgtcga tgatggcgcg ctccatcatg tctgtgggtg gggcagccca tcaggtgccg 420
   ggcaggccct ctcgtcctca cacctgtcct cccccatgaa tcctccagcc ggtcttccgg 480
   gcctgctcct caccctggat ccagacgaag ccgtagagga agtagaagcg gccgccagta 540
   ttgggcccag gccgccagta gg 562
<210> 2
   <211> 562
   <212> DNA
   <213> Homo sapiens
<400> 2
   ttcatcacct gcgggtgggc caggggcttg gggcaggccc cggggaggac gccgcccctc 60
   cctgccagcc cgcgcctcca gggagagtcc cggcccgcgc acctccttga gccggtgctc 120
   cttctccgcc acgatgtgct ggatggtcat ggccacggag tagacccagg agatcaccat 180
   gcacagcggc atcatgtgct caatgacaaa caggaagctg cggggaggcc gcgctcaggc 240
   gccactcagc cccagcccca gccccagccc cgggcgccca gcactcactc atcgcgtgtg 300
   tagcaggggt aggggaacat ctgcacgtag ctgcctggct ccaccacgtc gtgccccaca 360
   aaagtgtcga tgatggcgcg ctccatcatg tctgtgggtg gggcagccca tcaggtgccg 420
   ggcaggccct ctcgtcctca cacctgtcct cccccatgaa tcctccagcc ggtcttccgg 480
   gcctgctcct caccctggat ccagacgaag ccgtagagga agtagaagcg gccgccagta 540
   ttgggcccag gccgccagta gg 562
<210> 3
   <211> 400
   <212> DNA
   <213> Homo sapiens
<400> 3
   gggcccaata ctggcggccg cttctacttc ctctacggct tcgtctggat ccagggtgag 60
   gagcaggccc ggaagaccgg ctggaggatt catgggggag gacaggtgtg aggacgagag 120
   ggcctgcccg gcacctgatg gctgccccca cccacagaca tgatggagcg cgccatcatc 180
   gacacttttg tggggcacga tgtggtggag ccaggcagct acgtgcagat gttcccctac 240
   ccctgctaca cacgcgatga gtgagtgctg ggcgcccggg gctggggctg gggctggggc 300
   tgagtggcgc ctgagcgcgg cctccccgca gcttcctgtt tgtcattgag cacatgatgc 360
   cgctgtgcat ggtgatctcc tgggtctact ccgtggccat 400
<210> 4
   <211> 400
   <212> DNA
   <213> Homo sapiens
<400> 4
   gggcccaata ctggcggccg cttctacttc ctctacggct tcgtctggat ccagggtgag 60
   gagcaggccc ggaagaccgg ctggaggatt catgggggag gacaggtgtg aggacgagag 120
   ggcctgcccg gcacctgatg gctgccccca cccacagaca tgatggagcg cgccatcatc 180
   gacacttttg tggggcacga cgtggtggag ccaggcagct acgtgcagat gttcccctac 240
   ccctgctaca cacgcgatga gtgagtgctg ggcgcccggg gctggggctg gggctggggc 300
   tgagtggcgc ctgagcgcgg cctccccgca gcttcctgtt tgtcattgag cacatgatgc 360
   cgctgtgcat ggtgatctcc tgggtctact ccgtggccat 400
<210> 5
   <211> 8154
   <212> DNA
   <213> Homo sapiens
<400> 5
<210> 6
   <211> 16
   <212> DNA
   <213> Homo sapiens
<400> 6
   agcgcgccat catcga 16
<210> 7
   <211> 20
   <212> DNA
   <213> Homo sapiens
<400> 7
   cactcatcgc gtgtgtagca 20
<210> 8
   <211> 15
   <212> DNA
   <213> Homo sapiens
<400> 8
   cacgatgtgg tggag 15
<210> 9
   <211> 14
   <212> DNA
   <213> Homo sapiens
<400> 9
   cacgacgtgg tgga 14